# FASCICULE DE BREVET EUROPEEN

(11) **EP 1 461 003 B1**
(45) Date de publication et mention de la délivrance du brevet: **09.12.2009**
(21) Numéro de dépôt: 02804611.8
(22) Date de dépôt: 12.12.2002
(51) Int. Cl.: A61K 8/895, A61K 8/04, A61K 8/73, A61Q 5/02

(54) **UTILISATION D'UN COMPLEXE ELASTOMERE SILICONE POUR LA FABRICATION D'UN SHAMPOOING SEC SOUS FORME D'AEROSOL**
VERWENDUNG EINES SILIKONISIERTEN ELASTOMEREN KOMPLEXES ZUR HERSTELLUNG EINES TROCKENEN AEROSOL-SHAMPOOS
USE OF A SILICONIZED ELASTOMERIC COMPLEX FOR MAKING A DRY AEROSOL SHAMPOO

(30) Priorité: 13.12.2001 FR 0116104
(43) Date de publication de la demande: 29.09.2004
(73) Titulaire: Pierre Fabre Dermo-Cosmétique, 92100 Boulogne-Billancourt (FR)
(72) Inventeur: JEANJEAN, Michel, F-31320 Castanet-Tolosan (FR); SENEGAS, Nadine, F-31320 Castanet Tolosan (FR)
(74) Mandataire: Warcoin, Jacques
(86) Numéro de dépôt international: PCT/FR2002/004311
(87) Numéro de publication internationale: WO 2003/049711

(56) Documents cités:
- FR-A- 2 721 507
- Korean Patent Abstracts; Korean Industrial Property Office, 2001 XP002220204 & KR 265 240 B (HANKOOK COSMETICS CO LTD) 1 novembre 2000 (2000-11-01)

## Description

La présente invention concerne l'utilisation d'un complexe élastomère siliconé réticulé Diméthicone/Vinyldiméthicone, pour la fabrication d'un shampooing sec conditionné sous forme d'aérosol vectorisé par au moins un gaz propulseur hydrocarboné.

Les shampooings secs existent depuis de nombreuses années, soit sous forme poudreuse, soit sous forme d'aérosol. Cette dernière forme est la plus utilisée car elle offre l'avantage d'être plus pratique d'emploi, et d'assurer ainsi une meilleure répartition du produit sur la chevelure.

Les shampooings secs permettent d'éliminer l'excès de sébum d'une façon rapide, non contraignante et sans mouiller la chevelure. Ils agissent en captant le sébum par absorption grâce à des poudres choisies pour leurs qualités sébophiles, l'élimination des poudres s'effectuant par simple brossage.

Dans l'art antérieur, ces poudres, prises isolément ou en association, sont d'origine minérale, organique ou synthétique. A titre d'exemples de poudres ou de mélanges de poudres dégraissantes, peuvent ainsi être cités l'amidon de maïs modifié (FR 2 721 507) l'amidon de riz (FR 1 599 256), les poudres végétales délipidées et décolorées (FR 2 472 934), les polyamides (FR 2 555 441), les lyophilisats de poudres végétales (FR 2 207 688), la chitine (US 4 035 267), ainsi que la poudre de lycopode (FR 1 578 989). Cependant, dans la pratique, les shampooings secs proposés ne donnent pas entière satisfaction. En particulier, deux inconvénients majeurs sont généralement rencontrés, à savoir un dégraissage insuffisant du cheveu par suite d'une trop faible affinité des poudres vis-à-vis du sébum, et une élimination incomplète des poudres lors du brossage donnant à la surface de la chevelure un aspect insatisfaisant.

En outre, l'utilisation des hydrocarbures comme gaz vecteurs dans les aérosols a entraîné des problèmes galéniques pour la mise en suspension des poudres et leur distribution en aérosols, en raison de la différence de densité entre les poudres et la phase propulseur liquide. En effet, la densité des mélanges courants d'hydrocarbures, voisine de 0,55, est souvent très inférieure à celles des poudres pouvant être sélectionnées pour leur pouvoir dégraissant des cheveux. Ces forts écarts de densité pouvant exister entre les deux phases peuvent ainsi entraîner des phénomènes de décantation, d'agrégation ou de tassement des particules préjudiciables au bon fonctionnement de l'aérosol.

Par conséquent, il existait un besoin de mettre au point une nouvelle composition pour shampooing sec conditionné sous forme d'aérosol qui offre à la fois une activité nettoyante optimale, une excellente élimination au moment du coiffage et des propriétés physiques qui la rendent parfaitement adaptée à la propulsion par un mélange d'hydrocarbures.

La présente invention vient combler ce besoin. La Demanderesse a ainsi découvert de manière surprenante que l'utilisation d'un complexe élastomère siliconé, réticulé Diméthicone/Vinyldiméthicone, pour la fabrication d'un shampooing sec conditionné sous forme d'aérosol, permettait d'obtenir un shampooing sec respectant l'ensemble des critères définis ci-dessus, à savoir le pouvoir dégraissant, la facilité d'élimination par brossage et la compatibilité avec la propulsion par un mélange d'hydrocarbures.

Le diméthicone/vinyldiméthicone crosspolymer est un agent d'absorption des huiles utilisé dans des compositions pour la peau (KR 00265240 B1). Ainsi, en ce qui concerne la propulsion, le shampooing sec selon la présente invention permet notamment de faire varier la quantité ou la qualité des poudres par rapport au volume du mélange gazeux propulseur et de diminuer de manière remarquable les phénomènes de décantation, de séparation et de tassement des particules dispersées dans la phase propulseur liquide. Il est ainsi plus facile d'obtenir une meilleure suspension particulaire dans le boîtier aérosol.

En outre, le shampooing sec préparé selon la présente invention permet également d'éviter les risques de tassement, donc de bouchage, par passage à travers la valve, dans la mesure où le complexe élastomère siliconé utilisé, notamment le polymère réticulé Diméthicone/Vinyldiméthicone, a la propriété de gélifier de façon particulièrement performante les propulseurs hydrocarbonés tels que les mélanges de butane, propane ou isobutane. Ainsi, selon la concentration en complexe élastomère siliconé, il sera possible d'obtenir une gélification partielle ou totale du mélange propulseur d'hydrocarbures et la nature rhéologique du gel obtenu rendra alors ledit gel tout à fait propre à la pulvérisation.

Par ailleurs, outre le pouvoir gélifiant obtenu sur les mélanges de gaz liquéfiés, l'incorporation d'un complexe siliconé tel que le polymère réticulé Diméthicone/Vinyldiméthicone présente deux avantages complémentaires :
- associé aux autres poudres, il favorise leur circulation à travers la valve et le diffuseur par effet lubrifiant dû à sa nature siliconée,
- il permet d'améliorer le passage de la brosse sur la chevelure, ainsi que l'élimination des autres poudres de la chevelure.

La présente invention a ainsi pour objet l'utilisation d'un complexe élastomère siliconé pour la fabrication d'un shampooing sec conditionné sous forme d'aérosol vectorisé par au moins un gaz propulseur hydrocarboné.

Le complexe élastomère siliconé est selon la présente invention le polymère réticulé Diméthicone/Vinyldiméthicone. Ce complexe est vendu notamment par la société DOW CORNING, sous le nom commercial DOW CORNING^{®} 9506 POWDER. Sous forme de poudre blanche, de densité très faible (inférieure à 0,20), ce complexe présente la particularité d'absorber les huiles ainsi qu'un certain nombre de solvants apolaires tels que des esters gras, des triglycérides naturels ou fractionnés, des silicones fluides, des paraffines ou encore des isoparaffines liquides classiques à chaînes inférieures à C₁₀. Ce polymère réticulé Diméthicone/Vinyldiméthicone permet également d'améliorer la qualité de diffusion des poudres, notamment la suspension des systèmes particulaires en milieu propulseur liquide, et ce polymère peut ainsi être avantageusement utilisé dans tout type d'aérosol.

Dans un mode de réalisation particulier de la présente invention, le shampooing sec est constitué d'une phase solide poudreuse (a) contenant ledit complexe élastomère et au moins une poudre micronisée et calibrée, et d'une phase liquide (b) contenant au moins un gaz propulseur hydrocarboné liquéfié.

Une caractéristique importante pour le bon fonctionnement de l'aérosol est la finesse particulaire. La poudre sera donc de préférence micronisée pour une parfaite circulation à travers tous les orifices du système mécanique de diffusion, c'est-à-dire la valve et le diffuseur. Le terme « micronisé » désignera, selon la présente invention, une poudre de granulométrie moyenne calibrée inférieure à environ 100 microns et de préférence inférieure à environ 50 microns. Le calibrage des poudres pourra être obtenu par broyage à sec, cryobroyage et tamisage. Il conviendra lors de ces opérations d'éliminer les particules trop fines, jugées par expérience trop difficiles à éliminer au brossage en raison d'une adhérence trop forte à la tige capillaire.

En outre, la finesse particulaire, qui est corrélée à la surface spécifique de la poudre, est aussi très importante en ce qui concerne le pouvoir couvrant de la poudre et par conséquent son efficacité en tant qu'agent nettoyant, en particulier à faible dose. Ainsi affinées, les matières pulvérulentes seront facilement véhiculables sous la forme de shampooing sec aérosol.

Avantageusement selon la présente invention, la poudre est choisie dans le groupe constitué par les amidons naturels, tels que les amidons de riz ou de maïs ; les amidons modifiés, tels que les amidons de riz phosphaté, ou les amidons Dry Flo^{®} de National Starch ; les argiles naturelles ; les argiles modifiées ; les silices absorbantes ; les poudres végétales broyées et calibrées, telles que les poudres de réglisse, de cacao, d'ortie, de noyaux, de rafles de maïs, ou encore de lycopode ; les β-cyclodextrines et leurs mélanges.

Avantageusement selon la présente invention, le gaz propulseur hydrocarboné est choisi dans le groupe constitué par le propane, le butane, le n-butane, l'isopentane, le DME et leurs mélanges, notamment les mélanges binaires ou ternaires.

Selon une caractéristique particulière de la présente invention, le complexe est présent à une concentration comprise entre 1 % et 10 % en poids par rapport au poids total du shampooing sec.

Avantageusement selon la présente invention, la phase liquide (b) contient en outre au moins un solvant organique présentant un bon niveau de volatilité ou ne laissant aucun résidu sur le cheveu. De manière encore plus avantageuse selon la présente invention, le solvant organique est choisi dans le groupe constitué par les silicones cycliques, les diméthicones de viscosité très basse, de préférence l'héxaméthyldisiloxane, les isoparaffines dont la chaîne carbonée est inférieure à C₁₀, et les esters gras à chaînes courtes, notamment les esters présentant des qualités cosmétiques en ce qui concerne leur application sur les cheveux.

Toutes sortes d'adaptations peuvent être effectuées à partir du shampooing sec de base préparé selon la présente invention, telles que l'incorporation de principes actifs, parfums, colorants, etc.

Les exemples suivants sont donnés à titre non limitatif et illustrent la présente invention.

### Exemple 1 : Intérêt d'associer le complexe élastomère siliconé aux autres poudres d'un aérosol en termes de propulsion :

Associé aux autres poudres, le polymère réticulé Diméthicone/Vinyldiméthicone favorise leur circulation à travers la valve et le diffuseur par effet lubrifiant dû à sa nature siliconée.

Des tests de vidanges effectués sur les deux lots de boîtiers suivants confirment l'intérêt technique de cette association :
- 10 boîtiers aérosols remplis à 150 ml au Butane 3.2 contenant 5 g d'amidon de maïs (Lot M),
- 10 boîtiers aérosols remplis à 150 ml au Butane 3.2 contenant 5 g d'amidon de maïs et 3 g de polymère réticulé Diméthicone/Vinyldiméthicone (Lot P).

Les essais de vidange, pour chaque lot, sont effectués selon la même technique :
- boîtiers tenus en position verticale,
- une seule agitation de chaque boîtier avant le démarrage de la diffusion.

Pour le lot P, 9 boîtiers sont vidangés totalement sans difficulté, 1 seul boîtier a manifesté un début de bouchage de valve aux ¾ de la vidange, nécessitant une agitation complémentaire pour le vider totalement de son contenu.

Pour le lot M, 3 aérosols ont été vidangés totalement, mais laissant un résidu de poudre supérieur à 30% en fond de boîtier. 7 aérosols ont manifesté un engorgement de la valve, nécessitant systématiquement une agitation pour poursuivre la vidange. L'un des boîtiers a présenté un bouchage irrémédiable au niveau du système de diffusion.

### Exemple 2 : Intérêt d'associer le complexe élastomère siliconé aux autres poudres d'un aérosol en termes de facilité d'élimination des poudres par brossage :

Lors d'essais sur chevelure, il a été remarqué que le polymère réticulé Diméthicone/Vinyldiméthicone permettait d'améliorer de façon évidente le passage de la brosse, ainsi que l'élimination des autres poudres de la chevelure. En effet, il est souvent constaté que le passage de la brosse est rendu difficile par l'absence d'effet glissant. La facilité d'élimination des poudres a été évaluée en « cabine de coiffage », sur des cheveux bruns, l'appréciation étant effectuée et cotée par deux personnes expertes dans le test psychosensoriel. Ces essais ont été conduits sur 10 sujets féminins présentant des cheveux gras : 8 cas de cheveux courts et 2 cas de cheveux longs.

Le test a été conduit sur ½ têtes, chaque personne étant en quelque sorte son propre témoin. Dans tous les cas, le côté traité avec l'association amidon de maïs/polymère réticulé Diméthicone/Vinyldiméthicone a montré une nette amélioration au passage de la brosse, donc une meilleure élimination de l'amidon dont la blancheur permet de caractériser de façon évidente l'insuffisance de ce paramètre.

### Exemple 3 : Formulation pour un shampooing sec en aérosol :

| *Phase solide poudreuse (a) :* | |
|---|---|
| - Polymère réticulé Diméthicone/Vinyldiméthicone | 4 à 6 g |
| - β-cyclodextrine micronisée | 0,1 à 3 g |
| - amidon de maïs modifié | 1 à 10 g |
| - amidon de riz phosphaté | 0,1 à 3 g |
| *Phase liquide (b) :* | |
| - éthanol | QS |
| - parfum | QS |
| - décaméthylcyclopentasiloxane | QS |
| - mélange propulseur à base d'hydrocarbures | QSP 150 ml |

## Revendications

1. Utilisation d'un polymère réticulé Diméthicone/Vinyldiméthicone pour la fabrication d'un shampooing sec conditionné sous forme d'aérosol vectorisé par au moins un gaz propulseur hydrocarboné.

2. Utilisation d'un polymère selon la revendication 1, **caractérisée en ce que** le shampooing sec est constitué d'une phase solide poudreuse (a) contenant ledit polymère et au moins une poudre micronisée et calibrée, et d'une phase liquide (b) contenant au moins un gaz propulseur hydrocarboné liquéfié.

3. Utilisation d'un polymère selon la revendication 2, **caractérisée en ce que** la poudre est choisie dans le groupe constitué par les amidons naturels, les amidons modifiés, les argiles naturelles, les argiles modifiées, les silices absorbantes, les poudres végétales broyées et calibrées, les β-cyclodextrines et leurs mélanges.

4. Utilisation d'un polymère selon la revendication 2 ou 3, **caractérisée en ce que** le gaz propulseur hydrocarboné est choisi dans le groupe constitué par le propane, le butane, le n-butane, l'isopentane, le DME et leurs mélanges.

5. Utilisation d'un polymère selon l'une quelconque des revendications 2 à 4, **caractérisée en ce que** le complexe est présent à une concentration comprise entre 1 % et 10 % en poids par rapport au poids total du shampooing sec.

6. Utilisation d'un polymère selon l'une quelconque des 2 à 5, **caractérisée en ce que** le shampooing sec est constitué :
d'une phase solide poudreuse (a) comprenant :
| | |
|---|---|
| - Polymère réticulé Diméthicone/Vinyldiméthicone | 4 à 6 g |
| - β-cyclodextrine micronisée | 0,1 à 3g |
| - amidon de maïs modifié | 1 à 10 g |
| - amidon de riz phosphaté | 0,1 à 3 g |
| et d'une phase liquide (b) comprenant : | |
| - éthanol | QS |
| - parfum | QS |
| - décaméthylcyclopentasiloxane | QS |
| - mélange propulseur à base d'hydrocarbures | QSP 150 ml |

7. Utilisation d'un polymère selon l'une quelconque des revendications 2 à 6, **caractérisée en ce que** la phase liquide (b) contient en outre au moins un solvant organique présentant un bon niveau de volatilité ou ne laissant aucun résidu sur le cheveu.

8. Utilisation d'un polymère selon la revendication 7, **caractérisée en ce que** le solvant organique est choisi dans le groupe constitué par les silicones cycliques, les diméthicones de viscosité très basse, les isoparaffines dont la chaîne carbonée est inférieure à C₁₀, et les esters gras à chaînes courtes.

## Claims

1. The use of a cross-linked dimethicone/vinyl dimethicone polymer for making a dry shampoo, packaged as an aerosol, borne by at least one hydrocarbon gas propellant.

2. The use of a polymer according to claim 1, **characterized in that** the dry shampoo consists of a powdery solid phase (a) containing said polymer and at least one micronized and calibrated powder, and of a liquid phase (b) containing at least one liquefied hydrocarbon gas propellant.

3. The use of a polymer according to claim 2, **characterized in that** the powder is selected from the group formed by natural starches, modified starches, natural clays, modified clays, absorbent silicas, milled and calibrated vegetable powders, β-cyclodextrins and mixtures thereof.

4. The use of a polymer according to claim 2 or 3, **characterized in that** the hydrocarbon gas propellant is selected from the group formed by propane, butane, n-butane, isopentane, DME and mixtures thereof.

5. The use of a polymer according to any of claims 2 to 4, **characterized in that** the complex is present at a concentration comprised between 1% and 10% by weight based on the total weight of the dry shampoo.

6. The use of a polymer according to any of claims 2 to 5, **characterized in that** the dry shampoo consists of:
a powdery solid phase (a) comprising:
| | |
|---|---|
| - crosslinked dimethicone/vinyl dimethicone polymer | 4-6 g |
| - micronized β-cyclodextrin | 0.1-3 g |
| - modified maize starch | 1-10 g |
| - rice starch phosphate | 0.1-3 g |
| and a liquid phase (b) comprising | |
| - ethanol | QS |
| - perfume | QS |
| - decamethylcyclopentasiloxane | QS |
| - propellant mixture based on hydrocarbons QSP | 150ml |

7. The use of a polymer according to any of claims 2 to 6, **characterized in that** the liquid phase (b) further contains at least one organic solvent having a good level of volatility or not leaving any residue on hair.

8. The use of a polymer according to claim 7, **characterized in that** the organic solvent is selected from the group formed by cyclic silicones, very low viscosity dimethicones, isoparaffins with a carbon chain of less than C₁₀, and short chain fatty esters.

## Patentansprüche

1. Verwendung eines retikulierten Dimethicon/Vinyldimethicon-Polymers zur Herstellung eines Trockenshampoons, das in Form eines durch mindestens ein Kohlenwasserstoff-Treibgas vektorisierten Aerosols konditioniert ist.

2. Verwendung eines Polymers nach Anspruch 1, **dadurch gekennzeichnet, dass** das Trockenshampoon von einer pulverförmigen festen Phase (a), die das Polymer und mindestens ein mikronisiertes und kalibriertes Pulver enthält, und von einer flüssigen Phase (b), die mindestens ein verflüssigtes Kohlenwasserstoff-Treibgas enthält, gebildet wird.

3. Verwendung eines Polymers nach Anspruch 2, **dadurch gekennzeichnet, dass** das Pulver aus der Gruppe ausgewählt ist, die von den natürlichen Stärken, den modifizierten Stärken, den natürlichen Tonen, den modifizierten Tonen, den absorbierenden Siliziumoxiden, den zerkleinerten und kalibrierten pflanzlichen Pulvern, den β-Cyclodextrinen und ihren Gemischen gebildet wird.

4. Verwendung eines Polymers nach Anspruch 2 oder 3, **dadurch gekennzeichnet, dass** das Kohlenwasserstoff-Treibgas aus der Gruppe ausgewählt ist, die von dem Propan, dem Butan, dem n-Butan, dem Isopentan, dem DME und ihren Gemischen gebildet wird.

5. Verwendung eines Polymers nach einem der Ansprüche 2 bis 4, **dadurch gekennzeichnet, dass** der Komplex in einer Konzentration zwischen 1 und 10 Gew.-% inklusive im Verhältnis zum Gesamtgewicht des Trockenshampoons vorhanden ist.

6. Verwendung eines Polymers nach einem der Ansprüche 2 bis 5, **dadurch gekennzeichnet, dass** das Trockenshampoon gebildet wird:
von einer pulverförmigen festen Phase (a), die umfasst:
| | |
|---|---|
| - retikuliertes Dimethicon/Vinylmethicon-Polymer | 4 bis 6 g |
| - mikronisiertes β-Cyclodextrin | 0,1 bis 3 g |
| - modifizierte Maisstärke | 1 bis 10 g |
| - phosphatierte Reisstärke | 0,1 bis 3 g |
und einer flüssigen Phase (b), die umfasst:
| | |
|---|---|
| - Ethanol | QS |
| - Duftstoff | QS |
| - Decamethylcyclopentasiloxan | QS |
| - Treibgemisch auf der Basis von Kohlenwasserstoffen | QS 150 ml |

7. Verwendung eines Polymers nach einem der Ansprüche 2 bis 6, **dadurch gekennzeichnet, dass** die flüssige Phase (b) weiterhin mindestens ein organisches Lösungsmittel enthält, das einen guten Flüchtigkeitsgrad aufweist oder keinen Rückstand auf dem Haar hinterlässt.

8. Verwendung eines Polymers nach Anspruch 7, **dadurch gekennzeichnet, dass** das organische Lösungsmittel aus der Gruppe ausgewählt ist, die von den zyklischen Silikonen, den Dimethiconen mit sehr niedriger Viskosität, den Isoparaffinen, deren Kohlenstoffkette unter C₁₀ ist und den kurzkettigen Fettestern gebildet wird.
